Europäisches Patentamt

European Patent Office

Office européen des brevets

(19)

(11)     EP 1 169 639 B1

## (12)     FASCICULE DE BREVET EUROPEEN

(45) Date de publication et mention
de la délivrance du brevet:
**28.12.2005   Bulletin 2005/52**

(21) Numéro de dépôt: **00917141.4**

(22) Date de dépôt: **06.04.2000**

(51) Int Cl.⁷: **G01N 33/03**

(86) Numéro de dépôt international:
**PCT/FR2000/000871**

(87) Numéro de publication internationale:
**WO 2000/062057 (19.10.2000 Gazette 2000/42)**

(54) **PROCEDE ET DISPOSITIF DE CONTROLE IN SITU D'UNE CUVE CONTENANT UNE HUILE OU GRAISSE DE FRITURE**

VERFAHREN UND VORRICHTUNG ZUR STEUERUNG "IN SITU" EINER FRITTEUSE

METHOD AND APPARATUS FOR CONTROLLING "IN SITU" A FRYER

(84) Etats contractants désignés:
**AT BE CH CY DE DK ES FI FR GB GR IE IT LI LU
MC NL PT SE**

(30) Priorité: **13.04.1999  FR 9904597**

(43) Date de publication de la demande:
**09.01.2002   Bulletin 2002/02**

(73) Titulaire: **Metatron
93220 Gagny (FR)**

(72) Inventeurs:
 • **PERNOT, Christian
 F-93220 Gagny (FR)**

 • **LE HENAFF, Denis
 F-93270 Sevran (FR)**

(74) Mandataire: **Santarelli
 14, avenue de la Grande Armée,
 B.P. 237
 75822 Paris Cedex 17 (FR)**

(56) Documents cités:
 **US-A- 5 239 258          US-A- 5 594 327
 US-A- 5 818 731**

EP 1 169 639 B1

## Description

L'invention concerne le contrôle in situ de la dégradation des huiles et graisses de friture (en pratique dans le domaine alimentaire).

Etat de la technique antérieure

Les huiles et graisses alimentaires utilisées dans les opérations de cuisson et de friture subissent une altération thermo-oxydative conduisant à une dégradation progressive de l'huile avec la formation de composés polaires.

En France, le décret 86-857 du 18 Juillet 1986, modifiant le décret du 11 Mars 1908, stipule que les graisses et huiles dont la teneur en composés polaires est supérieure à 25 % sont impropres à la consommation humaine et prévoit la fixation par arrêté d'une méthode analytique de référence pour déterminer la teneur en composés polaires.

L'actuelle méthode analytique de référence normalisée est une chromatographie liquide à pression atmosphérique suivie d'une évaporation de solvant et d'une pesée (durée 3 heures). Cette méthode est inapplicable pour un contrôle de routine.

Par ailleurs, une appréciation subjective, en surveillant le brunissement de l'huile, la viscosité, l'apparition de mousse ou la présence de fumées n'est pas fiable car ces critères peuvent apparaître bien après la limite des 25 %.

Des méthodes plus rapides ont été développées ou envisagées :

A - Méthodes basées sur des réactions colorimétriques :

- testeur « LRSM » de la société 3M : il consiste à tremper un papier réactif dans l'huile. Le critère définissant la fin d'utilisation d'une huile est le nombre de bandelettes du papier réactif ayant changé de couleur.
- testeur « OXYFRITEST » des laboratoires MERCK-CLEVENOT : une éprouvette est remplie d'huile à l'aide d'une seringue et l'on y ajoute quelques gouttes de réactif. Après quelques secondes, en fonction de la teneur en composés polaires la couleur est modifiée. La limite d'utilisation est définie par comparaison de la couleur obtenue avec une échelle de couleurs de référence.
- Brevet FR-2 513 765 (OIL PROCESS SYSTEMS INC.) pour: « Nécessaire et procédé pour le dosage des substances alcalines dans les matières grasses alimentaires » ; le dosage se fait à partir d'un mélange de matière grasse avec une solution de titrage contenant un solvant et un colorant.

B - Méthodes basées sur la mesure de la conductivité ou de la résistance spécifique :

- Brevet US - 5 239 258 (KAUFFMAN) pour : « Freshness and Stability Test Using Oxidative Degradation » ; le dosage se fait à partir de l'évolution du courant passant dans un échantillon, en fonction de la tension appliquée.
- Brevet US - 5 594 327 (SAGREDOS et al.) pour : « Method For Determining the Degree of Deterioration of Oils or Fats Used for Frying Foods » ; le dosage se fait sur des échantillons introduits dans une cellule de mesure où, sous une tension de 100V et à une température de 75 °C, la résistance ohmique spécifique du matériau constitutif de l'échantillon est mesurée.

C - Méthodes basées sur la mesure de la constante diélectrique:

Divers articles scientifiques ont déjà traité du problème de la dégradation des huiles, comme par exemple :

* « Changes in Dielectric Constant as a Measure of Frying Oil Deterioration », de C.W. FRITSCH et al., paru dans « Journal of The American Oil Chemists » Society, Vol. 56, August 1979,
* « Measurements of Frying Fat Deterioration. A Brief Review » de C. W. FRITSCH et al. paru dans JAOCS, March 1981.

Ces articles ont mis en évidence une forte corrélation entre la variation de la constante diélectrique et le pourcentage de composés polaires formés. Cela est mis à profit dans la demande de brevet DE - 30 06 696 5 (NORTHERN INSTRUMENTS CORP.) pour « Vorrichtung zum Messen dielektrischen Eigenschaften ». Le dispositif de mesure de caractéristiques diélectriques qui y est décrit est parfois désigné sous la désignation anglo-saxonne de « Food Oil Sensor ».

En pratique, seuls les testeurs de 3M et de MERCK-CLEVENOT sont utitisés. La durée d'un test est d'environ une minute par friteuse. Mais la fiabilité de ces testeurs reste faible car elle dépend de 3 facteurs difficilement maitrisables :

1°) conditions de stockage des réactifs : température, temps, lumière ambiante.
2°) leur mise en oeuvre :

- les papiers réactifs ne doivent pas être mis en contact avec d'autres objets et doivent être immergés pendant un temps précis -quelques secondes- dans l'huile homogénéisée.
- la quantité de réactif -quelques gouttes- doit être impérativement respectée.

3°) mesure et interprétation :

- elle doit être faite après un délai précis (une dizaine de secondes) et n'est valable que pendant un temps déterminé (une trentaine de secondes).
- les changements de couleur sont progressifs et demandent une certaine habitude pour être lus correctement.

En règle générale, l'ensemble des testeurs disponibles ou cités dans la littérature présentent tous l'inconvénient de procéder par échantillonnage, c'est à dire par prélèvements d'échantillons qui sont analysés en dehors de la masse de matière grasse en service. La fiabilité du contrôle dépend donc systématiquement de la périodicité et de la régularité des tests.

Bien entendu, cette fiabilité d'ensemble dépend en outre de la fiabilité des conséquences tirées des résultats des opérations de test.

Toutes les méthodes nécessitant une préparation préalable (chimiques, colorimétriques, ou même électrique - brevet US - 5 239 258) ne peuvent pas être utilisées en contrôle in situ et ne se prêtent guère à un contrôle fréquent (typiquement plusieurs fois au cours d'un cycle de chauffage).

Le brevet US n° 5 594 327 décrit une méthode de mesure de variation de la conductivité en utilisant une tension continue de 100 V, méthode inapplicable pour un contrôle dans une cuve de cuisson commerciale (problème de tension de sécurité).

Il faut noter à propos de ce dernier document qu'il mentionne, parmi d'autres techniques de contrôle, la mesure de la constante diélectrique du bain de friture, mais il juge cette méthode complexe et coûteuse en temps de contrôle, sans dire pourquoi. On peut toutefois faire les observations suivantes :

* *à propos de la complexité*

la composition de huiles et graisses de fritures évolue en permanence en fonction des besoins de la clientèle ou des prix d'achat des matières premières ; une huile peut comporter des graisses animales (suif par exemple), des graisses végétales (palme par exemple), un anti-oxydant *(Exxx),* et/ou un anti-moussant *(Eyyy).* Or la permittivité d'une telle huile, à 180 °C (température typique d'utilisation), ne figure dans aucune publication, y compris les spécifications techniques du fabricant, ni a fortiori

- sa dispersion lors de fabrications successives,
- sa variation lorsqu'elle renferme 25 % de composés polaires.

De plus, chaque groupe de restauration utilise une huile ou une graisse dont il a lui-même déterminé la composition.

* *à propos du coût en temps de contrôle*

le brevet ci-dessus cité se réfère sans doute au FOOD OIL SENSOR qui procède par échantillonnage, ce qui se révèle bien évidemment long et complexe.

Ce document D1 concerne un procédé et un appareil de mesure de la qualité d'une huile de cuisson par mesure ponctuelle de diverses propriétés, dont la capacitance, au sein d'une chambre recevant, pour chaque mesure, un échantillon d'huile au travers d'un filtre. Cette qualité est évaluée par comparaison, notamment, de la valeur mesurée de la capacitance à une valeur de référence stockée au préalable, en fonction du type de l'huile considérée (il est exclu de prendre de l'huile fraîche comme référence).

**Solution apportée**

L'invention a pour objet de pallier les inconvénients des solutions connues, en assurant un contrôle à la fois fiable et répétitif, et qui se prête à une automatisation en sorte de pouvoir notamment garantir les conséquences de la détection d'une quantité trop importante de composés polaires.

L'invention propose à cet effet un procédé de contrôle d'une cuve de friture contenant un bain d'huile ou de graisse de friture destiné à suivre une pluralité de cycles de chauffe, selon lequel :

* on installe à demeure dans une zone de la cuve adaptée à être immergée dans le bain une sonde capacitive dont l'espace diélectrique est constitué par une portion dudit bain, et, après remplissage de la cuve avec ce bain, on déclenche une procédure d'initialisation au cours de laquelle on mesure une valeur initiale d'une caractéristique représentative de la constante diélectrique de ce bain en conditions de chauffe, et on fixe un critère de mise hors service,
* de façon automatique, on détecté, de manière répétée au cours des cycles de chauffe suivants, une mesure de cette caractéristique et on déclenche une procédure de mise hors service lorsqu'il est détecté que le critère de mise hors service est satisfait par cette mesure.

Ainsi l'invention enseigne d'utiliser une sonde capacitive, en pratique à demeure, dans la cuve de cuisson. La sonde doit donc supporter une température moyenne de 180°C et sa construction doit satisfaire aux normes alimentaires et électriques ; en fait les contraintes pour la sonde peuvent s'exprimer comme suit :

- *températures élevées:* 180 °C avec un maximum de 210° C,
- *alimentarité*
  *robustesse :* nettoyages et filtrages quotidiens de la cuve par un personnel non qualifié.

Mais il apparaît qu'une sonde capacitive est tout à fait appropriée pour de telles conditions, compte tenu de sa simplicité (deux électrodes séparées par un espace diélectrique plongé dans le bain). D'autre part le fait de procéder par comparaison avec la valeur initiale de la caractéristique considérée, rend le procédé fiable, indépendamment d'un certain nombre de paramètres difficiles à contrôler, variant d'un bain d'huile à l'autre.

Dans les friteuses commerciales, de grande capacité, le capteur n'a pas besoin d'être miniaturisé. Il existe dans la cuve de cuisson une zone morte disponible pour implanter un capteur capacitif de dimensions suffisantes pour garantir sa robustesse et caractériser une partie substantielle du bain d'huile, bien représentative de celui-ci.

Il n'y a donc pas, contrairement à ce qui pouvait a priori être supposé, de difficultés particulières à installer à demeure (in situ) une sonde capacitive dans une cuve de friture.

Les avantages de la solution proposée, par rapport aux dispositifs connus sous la désignation « Food Oil Sensor » sont ainsi :

- contrôle répétitif in situ de l'huile dans la cuve de cuisson
- possibilité de contrôle automatique, sans intervention humaine (ce procédé est en effet aisément automatisable, au moyen d'asservissements).

Le procédé de l'invention peut mettre en oeuvre diverses dispositions particulières avantageuses, prises isolément ou en combinaison.

La détection des mesures est avantageusement périodique ; si l'on mesure la valeur instantanée de ladite caractéristique (ou si on fait une moyenne de telles valeurs instantanées) au moins une fois par heure (c'est un ordre de grandeur correspondant à plusieurs mesures par cycle de chauffe), on peut sans difficulté dire que le contrôle est continu compte tenu de la vitesse de dégradation des bains d'huile (leur durée de vie opérationnelle est en effet typiquement supérieure à 60 heures).

Une manière simple de procéder- est de mesurer ladite caractéristique par excitation d'un circuit oscillant dont fait partie la sonde capacitive.

Diverses caractéristiques du circuit oscillant peuvent être utilisées. De manière simple on mesure la période d'oscillation, c'est à dire que l'on mesure ladite caractéristique par excitation d'un circuit oscillant dont fait partie la sonde capacitive, et ladite caractéristique que l'on mesure est la période d'oscillation du circuit oscillant.

La mesure que l'on détecte peut être la moyenne de plusieurs mesures successives, ce qui a pour intérêt un effet de lissage.

Le critère de mise hors service fait de préférence intervenir une comparaison à la valeur initiale de la caractéristique.

Le fait de comparer la mesure à la valeur initiale de la caractéristique à laquelle on a choisi de s'intéresser peut consister à en faire le rapport. Mais de manière particulièrement simple, le critère de mise hors service est le franchissement d'un seuil de consigne par la différence entre la mesure "instantanée" et la valeur initiale de ladite caractéristique. En variante, ce critère de mise hors service peut aussi être défini à partir d'une variation absolue de cette mesure de la caractéristique.

Pour permettre à l'utilisateur de prendre à l'avance des dispositions pour remplacer un bain dont la fin de vie est proche, on fixe avantageusement, en outre, un critère d'alerte (celui-ci peut ou non être défini en fonction de la valeur initiale de la caractéristique), et on déclenche une procédure d'alerte (elle peut n'être que sonore ou visuelle, sans influer sur la marche de la cuve) lorsqu'il est détecté que ce critère d'alerte est satisfait.

De manière préférée, lorsque le critère de mise hors service est fixé par la différence entre les valeurs initiale et instantanée, on fixe avantageusement le critère d'alerte, en fonction de la valeur initiale de la caractéristique, par un seuil de différence qui est inférieur à la différence définissant le critère de mise hors service, et on déclenche une procédure d'alerte lorsque ce seuil intermédiaire est franchi.

Pour minimiser le caractère éventuellement brutal de la mise en oeuvre du procédé de l'invention, surtout lorsque celle-ci est automatisée on inhibe avantageusement la procédure de mise hors service jusqu'au cycle de chauffe suivant celui au cours duquel le critère de mise hors service a été détecté. Il n'y a donc aucune difficulté à terminer normalement le cycle de chauffe en cours, ce qui évite toute interruption intempestive de la fourniture des produits frits dans la cuve.

La procédure de mise hors service peut consister en un arrêt du cycle de chauffe qui suit celui au cours duquel le critère de mise hors service a été détecté, après un temps prédéterminé (éventuellement nul) destiné à permettre de détecter l'accomplissement, ou non, de manoeuvres de remplacement du bain et de saisie des données nécessaires au suivi de ce nouveau bain.

On comprend aisément que la fiabilité du contrôle dépend de l'élimination des causes possibles de mise hors service intempestive. C'est pourquoi, de manière avantageuse on ne tient compte que des mesures de la caractéristique faites lorsque la température du bain est au moins égale à un seuil ; c'est ainsi que, de manière préférée, on détecte en outre une caractéristique représentative de la température du bain, et on inhibe les mesures de la caractéristique ou le contrôle du critère de mise hors service lorsque la température du bain est inférieure à un seuil minimum de température (si la mesure consiste à prendre la moyenne de plusieurs saisies, il faut donc que chacune de ces saisies soit faite à une température supérieure au seuil).

De manière à pouvoir profiter des expériences passées, et de manière à pouvoir assurer un contrôle de la cuve sur une longue durée, y compris lors de changement de bain, on mémorise de préférence des données concernant des bains précédents. De manière avantageuse :

- lorsqu'on détecte que, après un temps prédéterminé après le début d'un cycle de chauffe suivant un cycle de chauffe au cours duquel le critère de mise hors service a été constaté, il n'y a pas eu de procédure d'initialisation, on provoque l'interruption du cycle de chauffe. Cela traduit en effet une anomalie possible de comportement de l'utilisateur.

- on inhibe toute procédure d'initialisation déclenchée au-delà d'un délai prédéterminé après le début d'un cycle de chauffe suivant un cycle de chauffe au cours duquel le critère de mise hors service a été constaté. Cela évite toute tentative de vouloir prolonger indûment la durée de vie d'un bain en prenant une nouvelle valeur d'initialisation, sans changer de bain.

- on définit la procédure d'initialisation en fonction d'au moins l'une des procédures d'initialisation des bains précédents mémorisées, ce qui permet de profiter des observations concernant le comportement des bains précédents, tels qu'ils peuvent par exemple être définis a posteriori par mesure précise de la teneur en composés polaires.

La procédure d'initialisation n'est avantageusement déclenchable qu'à partir d'un boîtier indépendant de la cuve. Il est donc possible de ne confier ce boîtier qu'à un responsable, et de fixer ainsi de manière claire les responsabilités dans le remplacement, ou non, des bains arrivés en fin de vie.

L'invention propose également un dispositif de contrôle automatique d'une cuve de friture contenant un bain d'huile ou de graisse de friture destiné à suivre une pluralité de cycles de chauffe, adapté à la mise en oeuvre du procédé, comportant :

* une sonde capacitive installée à demeure dans une zone de la cuve adaptée à être immergée dans le bain

* une unité de mesure et de traitement connectée à la sonde et à l'alimentation électrique de la cuve, et adaptée à mesurer des valeurs d'une caractéristique représentative de la constante diélectrique de ce bain en conditions de chauffe, et conçue en sorte :

  - de mémoriser, en réponse à un ordre d'initialisation, une mesure initiale de cette caractéristique et un critère de mise hors service, et

  - de détecter, de manière répétée au cours des cycles de chauffe du bain, une mesure de cette caractéristique, et de déclencher une procédure de mise hors service lorsqu'il est détecté que le critère de mise hors service est satisfait par cette mesure de la caractéristique,

**\*** une unité d'initialisation adaptée à être connectée à l'unité de mesure et de traitement et à lui transmettre un ordre d'initialisation et des données d'initialisation concernant le critère de mise hors service.

Par analogie avec les commentaires qui précèdent à propos du procédé, selon des enseignements préférés de l'invention, éventuellement combinés :

- l'unité de mesure et de traitement est conçue en sorte de mesurer ladite caractéristique au moins une fois par heure (c'est-à-dire plusieurs fois par cycle de chauffe).
- la sonde est montée dans un circuit oscillant, et l'unité de mesure et de traitement est conçue en sorte de mesurer la valeur instantanée d'une caractéristique de ce circuit oscillant.
- ladite caractéristique est la période d'oscillation du circuit oscillant.
- la mesure que l'on détecte est une moyenne de plusieurs mesures successives.
- l'unité de mesure et de traitement est conçue en sorte de pouvoir détecter le franchissement d'un seuil de consigne défini par l'unité d'initialisation au sein des données concernant le critère de mise hors service, par la différence entre la mesure et la valeur initiale de ladite caractéristique.
- l'unité de mesure et de traitement est en outre conçue en sorte de mémoriser un critère d'alerte (par exemple en fonction de la valeur initiale de la caractéristique). défini par l'unité d'initialisation, et de déclencher une procédure d'alerte lorsqu'il est détecté que ce critère d'alerte est satisfait par la mesure de la caractéristique.
- l'unité de mesure et de traitement est conçue en sorte d'inhiber la procédure de mise hors service jusqu'au cycle de chauffe suivant celui au cours duquel le critère de mise hors service a été détecté.
- la procédure de mise hors service consiste en un arrêt, par coupure de l'alimentation de la cuve, du cycle de chauffe qui suit celui au cours duquel le critère de mise hors service a été détecté, après un temps prédéterminé.
- ce dispositif comporte en outre un élément détecteur pour mesurer une caractéristique représentative de la température du bain, et l'unité de mesure et de traitement est connectée à cet élément détecteur et est conçue en sorte d'inhiber les mesures de la caractéristique ou le contrôle du critère de mise hors service lorsque la température du bain est inférieure à un seuil minimum de température.
- l'unité de mesure et de traitement est en outre conçue en sorte de mémoriser des données concernant des bains précédents.
- l'unité de mesure et de traitement est conçue en sorte de pouvoir détecter que, après un temps prédéterminé après le début d'un cycle de chauffe suivant un cycle de chauffe au cours duquel le critère de mise hors service a été constaté, il n'y a pas eu de transmission d'ordre d'initialisation ou de données depuis l'unité d'initialisation, et de pouvoir alors provoquer l'interruption du cycle de chauffe par coupure de l'alimentation de la cuve.
- l'unité de mesure et de traitement est en outre conçue en sorte d'inhiber tout ordre ou donnée d'initialisation en provenance de l'unité d'initialisation, au-delà d'un délai prédéterminé après le début d'un cycle de chauffe suivant un cycle de chauffe au cours duquel le critère de mise hors service a été constaté.
- l'unité d'initialisation est conçue en sorte de définir les données d'initialisation en fonction des données d'initialisation et de données échangées avec l'unité de mesure et de traitement pour l'un au moins des bains précédents.
- l'unité d'initialisation est connectable de façon amovible à l'unité de mesure et de traitement. -

En outre, pour bien dissocier les fonctions de mesure et de traitement, d'une part, et de commande (par le biais de l'ordre d'initialisation et du choix des données d'initialisation), de manière préférée, l'unité de mesure et de traitement est fixée à la cuve, et l'unité d'initialisation est contenue dans un boîtier amovible.

Dans le cas d'une friteuse électrique, la sonde capacitive est fixée de préférence dans la cuve, en dessous de résistances dont la cuve est munie pour le chauffage du bain. Il s'agit en effet d'un endroit disponible pour l'implantation de la sonde, et en outre permettant une fixation aisée de celle-ci, et une connexion aisée avec l'extérieur.

De manière particulièrement simple, la sonde capacitive comporte deux électrodes dont l'une est au potentiel constant de la cuve.

Description des dessins:

Des objets, caractéristiques et avantages de l'invention ressortent de la description qui suit, donnée à titre d'exemple illustratif non limitatif, en regard des dessins annexés sur lesquels :

- la figure 1 est un schéma général d'un dispositif de contrôle selon l'invention, ou contrôleur, comportant une sonde capacitive,
- la figure 2 est une vue de dessus de la cuve de cuisson d'une friteuse électrique, dans laquelle est implantée la sonde,
- la figure 3 en est une vue en coupe verticale,

- la figure 4 est une vue latérale de cette sonde de mesure,
- la figure 5 est une vue de dessus de ladite sonde de mesure,
- la figure 6 est une vue en perspective du boîtier d'analyse du contrôleur de la figure 1,
- la figure 7 est une vue en perspective du module d'interface du contrôleur de la figure 1,
- la figure 8 est une vue en perspective du boîtier amovible de contrôle de la figure 1,
- la figure 9 est le schéma de principe d'un exemple de circuit oscillant comportant la sonde de mesure, et
- la figure 10 est un organigramme simplifié du procédé de l'invention.

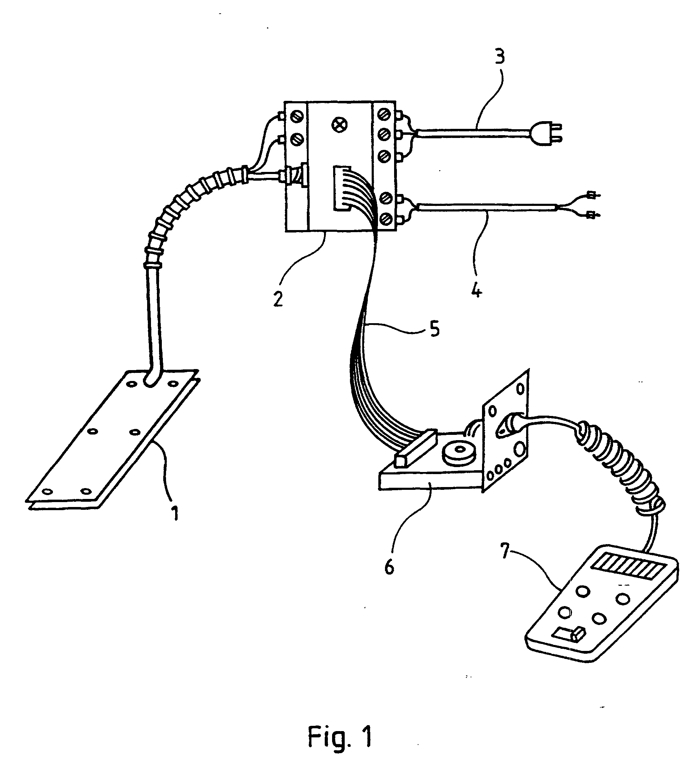

Les figures 1 à 9 représentent, à titre d'exemple illustratif non limitatif, un dispositif de contrôle d'une cuve de friture, en fonction de l'état du bain de matière grasse qu'il contient, qui comporte divers sous-ensembles :

* une sonde de mesure 1 destinée à être placée dans la cuve de cuisson,
* un boîtier d'analyse 2 destiné à être fixé au plus près de la sonde,
* un module d'interface 6 destiné à être fixé à l'avant de la friteuse, côté service, accessible facilement par le personnel,
* un boîtier amovible 7, connectable au module d'interface,
* un cordon d'alimentation secteur 3,
* un cordon 4 pour le contact d'asservissement de la friteuse,
* un câble multiconducteurs 5 de liaison entre le boîtier d'analyse et le module d'interface.

A propos de la sonde de mesure 1.

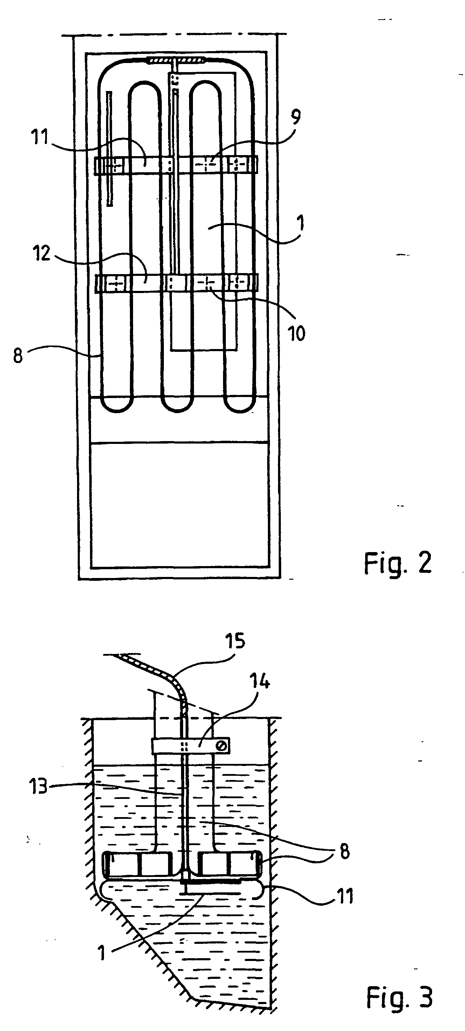

*1° Implantation dans la cuve de cuisson de la friteuse électrique (figures 2 et 3)*

La sonde 1 est placée sous une résistance de chauffe 8 en deux branches et est fixée par deux vis 9 et 10 aux deux barreaux 11 et 12 de maintien des résistances.
Des fils de mesures sont logés dans un tube 13 qui remonte le long de la paroi arrière de la cuve de cuisson. Le tube 13 est maintenu entre les deux branches de la résistance par un clips 14, ce qui lui assure une protection mécanique.
Une gaine inox souple 15 prolonge le tube 13 pour amener les fils de mesure au boîtier d'analyse 2 avec la protection nécessaire.

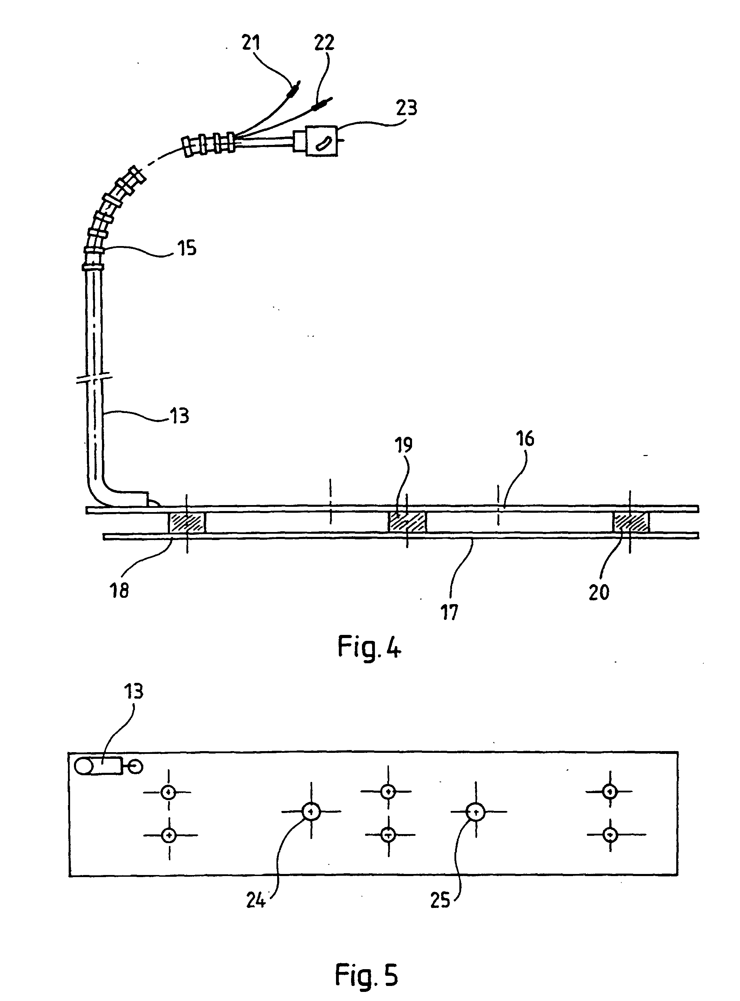

*2° Réalisation (figures 4 et 5)*

La sonde 1 est constituée de

- une électrode supérieure 16 : par exemple une plaque d'inox de 270 x 60 mm épaisseur 3 mm,
- une électrode inférieure 17 : par exemple une plaque d'inox de 270 x 60 mm épaisseur 2 mm,
- des isolateurs par exemple trois isolateurs 18, 19, 20, en téflon d'épaisseur 8,4 mm),
- un tube 13 en inox, par exemple de diamètre 6 mm, qui protège les fils de mesure dans la cuve de cuisson,
- un thermocouple ayant des sorties 21, 22 et dont la partie active se trouve de préférence au niveau de l'électrode 16, à l'intérieur du tube 13,
- une gaine souple inox 15 de protection des fils de mesure entre le tube 13 et le boîtier d'analyse 2,
- un connecteur 23 de raccordement des électrodes au boîtier d'analyse 2,
- deux trous taraudés 24, 25 pour la fixation de la sonde sur les barreaux 11 et 12.

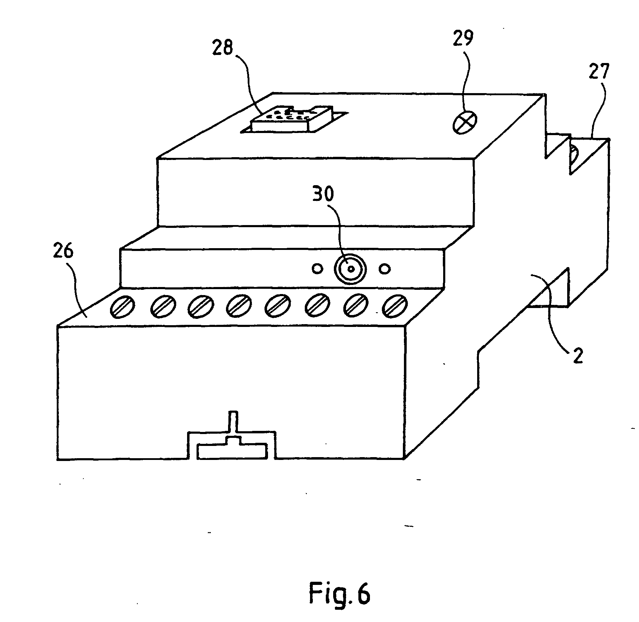

A propos du boîtier d'analyse 2 (figure 6)

Il est fixé à l'intérieur du châssis de la friteuse, à l'arrière, de telle sorte que la liaison avec la sonde soit la plus courte possible. A l'intérieur sont par exemple logés :

- une alimentation basse tension isolée galvaniquement du secteur,
- un circuit de détection du sens de raccordement du secteur,
- un générateur de signaux périodiques,
- des circuits de mesure et de contrôle de la fréquence comportant des éléments de mémorisation de valeurs antérieures, et
- un circuit de mesure de la température de l'huile.

Sur l'extérieur du boîtier sont avantageusement implantés :

- des bomiers à vis 26 et 27 pour le raccordement du thermocouple 21 et 22, du cordon d'alimentation 3 et du cordon d'asservissement de la friteuse 4,
- un connecteur 28 pour le raccordement du câble de liaison 5 entre le boîtier d'analyse 2 et le boîtier amovible 7,
- un voyant de présence tension secteur 29, et
- un embase de raccordement 30 pour le connecteur 23 ; ce connecteur et cette embase peuvent être supprimés, les électrodes de la sonde se raccordant directement aux bomiers à vis 26 et 27.

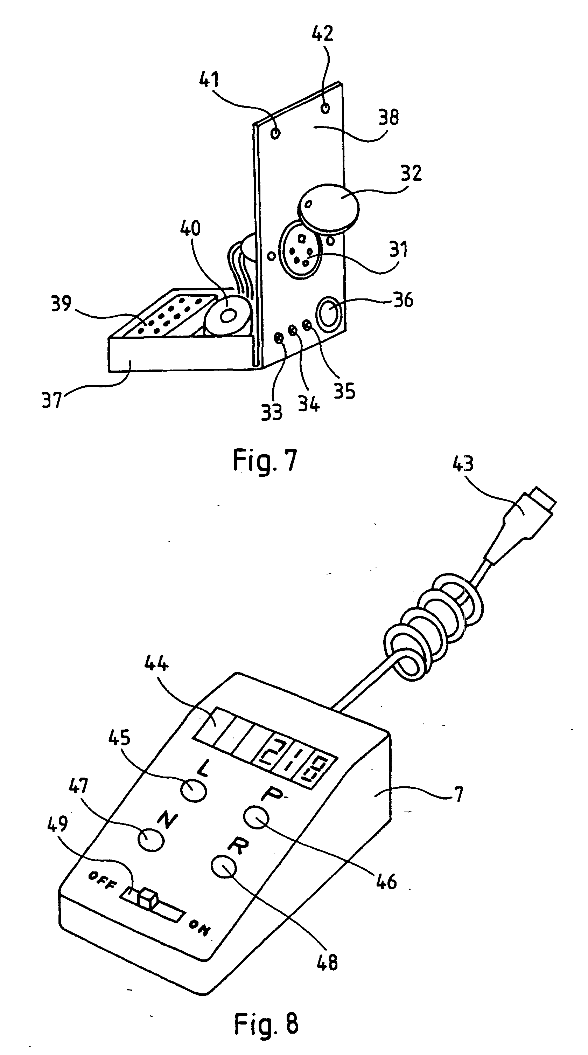

A propos du module d'interface 6 (figure 7)

Il est de préférence disposé à l'avant de la friteuse, derrière la porte d'accès aux équipements. Il est ici constitué d'une équerre en inox 38 sur laquelle sont implantés

- une embase femelle 31 pour la liaison avec la boîtier amovible 7,
- un cache pivotant 32 assurant une protection de l'embase 31,
- un voyant vert 33,
- un voyant jaune 34,
- un voyant rouge 35,
- un bouton poussoir 36,
- un boîtier plastique 37 protégeant le circuit imprimé de raccordement,
- une embase 39 pour le raccordement du câble 5, et
- un klaxon ou avertisseur sonore ou autre 40.

Le module d'interface se fixe sur le châssis de la friteuse grâce à deux trous 41 et 42.

A propos du boîtier amovible 7 (figure 8)

Il se raccorde au module d'interface 6 par un câble souple muni d'un connecteur 43. Il comprend :

- un afficheur à 6 digits 44,
- une touche L 45 pour la lecture de la mémoire du boîtier d'analyse 2,
- une touche P 46 pour la programmation du boîtier d'analyse 2,
- une touche N 47 pour visualiser différentes valeurs mémorisées,
- une touche R 48 pour régler les consignes du boîtier d'analyse 2,
- un interrupteur 49 pour la mise en, ou hors, service du boîtier amovible.

Le dispositif de contrôle peut être analysé comme comportant une sonde de mesure 1, une unité de mesure et de traitement (circuits d'analyse du boîtier 2 complétés par les éléments du module 6) et une unité d'initialisation (boîtier amovible 7).

**FONCTIONNEMENT**

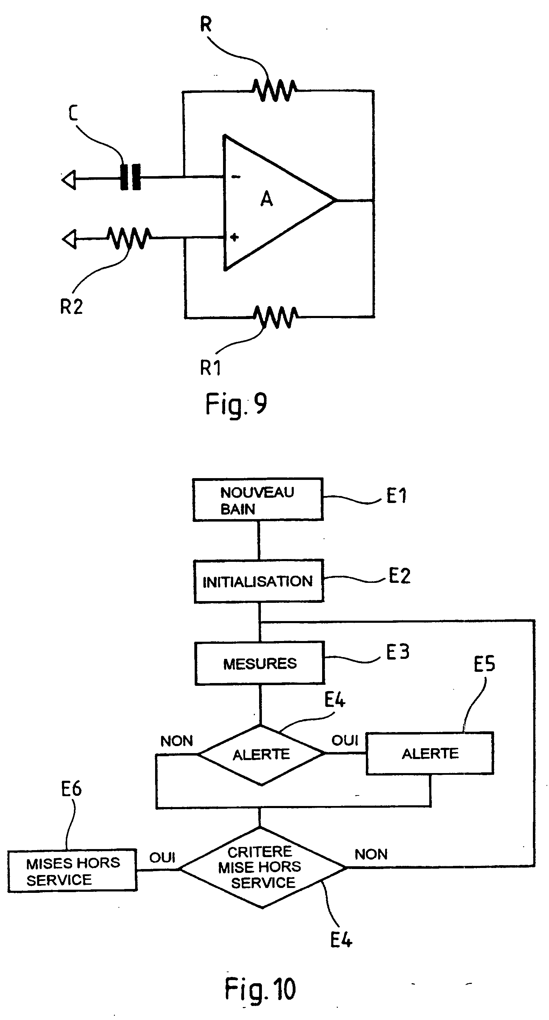

De manière générale, le procédé de l'invention comporte les étapes suivantes, schématisées à la figure 10 :

étape E1 : mise en place d'un nouveau bain ;
étape E2 : déclenchement d'une procédure d'initialisation, au cours de laquelle on mesure une valeur initiale d'une caractéristique représentative de la constante diélectrique de ce bain en condition de chauffe et on fixe un critère de mise hors service ainsi que, éventuellement, un critère d'alerte ;
étape E3 : détection, de manière répétée, d'une mesure de cette caractéristique ;
étape E4 : surveillance du critère de mise hors service et éventuellement du critère d'alerte ;
étape E5 : déclenchement de la procédure d'alerte lorsque le critère d'alerte est détecté, toute en répétant l'étape E3 ;
étape E6 : déclenchement de la procédure de mise hors service lorsque le critère de mise hors service est détecté.

Le contrôle est effectué dans l'exemple considéré en mesurant périodiquement la fréquence (ou' la période) du générateur et en comparant cette valeur à des fréquences $F_{80\%}$ et $F_{usée}$ calculées par le boîtier d'analyse au début de l'utilisation du bain considéré à partir d'une consigne de dégradation programmée transmise par le boîtier 7.

Le bain d'huile ou de graisse n'est pas homogène et présente des gradients de température. Pour utiliser de façon efficace les tests chimiques, il est recommandé, avant de faire un test, d'homogénéiser le bain en le remuant, à l'aide d'une louche par exemple, ce qui, en pratique n'est jamais fait.

La fréquence du contrôle résulte d'un compromis entre la vitesse moyenne de dégradation du bain et la fiabilité des mesures, compte tenu de la remarque ci-dessus.

La procédure utilisée par le contrôleur pour optimiser la fiabilité des mesures est par exemple la suivante :

* comptage pendant 5 secondes du nombre de périodes de l'oscillateur = n1,
* 10 secondes après : second comptage= n2,
* calcul et mémorisation à partir de ces deux mesures d'une première valeur moyenne : M1 = (n1 + n2) / 2.

Cette séquence est répétée toutes les 7 minutes 30 secondes.

La valeur prise en compte pour tester la qualité de l'huile est la moyenne de 4 valeurs successives M1, ... M4, à condition que la température du bain soit restée supérieure à la température de consigne durant cette période. Dans le cas contraire, la procédure est réinitialisée.

Le bain est donc testé régulièrement toutes les demi-heures. La durée moyenne d'utilisation d'un bain est de 72 heures (6 jours) ce qui entraîne systématiquement 144 tests, à comparer au 5 tests supposés être faits actuellement.

Cette différence permet de dire que le contrôle est continu. Ce caractère continu est d'autant plus réel que, si une mesure est saisie toutes les demi-heures, elle découle de paquets de mesures instantanées faites toutes les 7 minutes 30 secondes.

En principe les modalités de saisie des mesures font de préférence intervenir la moyenne de plusieurs mesures successives faites sur une durée suffisamment longue pour niveler les écarts dus à la nature hétérogène et aux mouvements de convection du bain, mais suffisamment courte par rapport à la cinétique de dégradation de ce bain pour avoir des valeurs successives proches les unes des autres, assurant une quasi-continuité de suivi de l'état du bain.

A chaque utilisation d'une huile de composition nouvelle, les consignes à programmer sont communiquées à l'utilisateur, par exemple par le fournisseur. En variante, l'utilisateur les déduit des suivis des bains antérieurs.

Après détection d'une usure de 100 %, l'huile doit être changée et le boîtier d'analyse réinitialisé, par le boîtier 7. Cette réinitialisation ne pouvant être faite que par le responsable ayant accès au boîtier amovible 7, les risques d'utilisation d'un bain au-delà des limites autorisées sont minimisés. Le paramétrage du boîtier d'analyse ne peut pas être fait sans le boîtier amovible, ce qui limite toute tentative de contournement du contrôle.

Les critères de mise hors service (et d'alerte, s'il en existe) peuvent être définis à partir d'une variation absolue de la caractéristique (la période dans l'exemple considéré) mais peuvent aussi être définis à partir de la valeur initiale de cette caractéristique.

*Début du contrôle d'un bain*

Au début de l'utilisation d'un nouveau bain d'huile, le boîtier d'analyse doit être réinitialisé. Cela consiste à raccorder le boîtier amovible au module d'interface et appuyer 2 fois sur la touche P. L'affichage du module d'interface passe alors de rouge clignotant à vert et jaune clignotants (par exemple pendant 30 mn) et la friteuse est mise en service. Le contrôle commence réellement lorsque le voyant vert seul est allumé, indiquant qu'une première mesure de la fréquence d'initialisation est faite.

*Etats transitoires*

Lorsque les voyants vert et jaune sont allumés simultanément, le contrôle est inhibé, l'huile n'ayant pas atteint la température de cuisson (mise en chauffe ou arrêt temporaire), typiquement de l'ordre de 180° C (on peut par exemple fixer un seuil de 175° C). Lorsque le voyant rouge s'allume en fixe, l'huile est proche de la fin d'utilisation (usée à 80 % environ), avertissant d'un changement proche. Il est bien sûr possible de se passer d'une telle procédure d'alerte.

*Fin d'utilisation du bain*

L'huile doit être changée lorsque le voyant rouge clignote et que l'avertisseur sonore est mis en service. Une pression sur le bouton poussoir arrête le klaxon. La friteuse reste en service jusqu'à ce qu'elle soit mise hors tension (continuité du service de restauration). A la mise sous tension suivante, l'affichage est immédiatement rouge clignotant et le klaxon est excité. Le boîtier d'analyse doit alors être réinitialisé, sinon, une demi-heure après, (temps nécessaire pour faire fondre la graisse et donc pouvoir l'évacuer), la friteuse est mise hors service, par l'ouverture du contact d'asservissement et le klaxon ne peut plus être arrêté par le bouton poussoir du module d'interface.

*Programmation des consignes*

Par sécurité, les 2 consignes ne sont avantageusement programmables que dans la demi-heure qui suit une réinitialisation (quant les voyants vert et jaune clignotent). Toute modification après cette période est détectée et mémorisée.

Consigne n°1 : température d'inhibition des mesures de 0 à 254 (°C),

Consigne n° 2 : valeur de période (en principe, c'est une variation) représentant le seuil de dégradation de l'huile de 0 à 255 (µs).

Pour cela, il faut raccorder le boîtier amovible au module d'interface puis

appuyer sur la touche P : entrée dans le mode de programmation,

appuyer sur la touche N : sélection de la consigne (1 ou 2),

appuyer sur la touche R : réglage de la valeur (incrémentation progressive),

appuyer sur la touche P : programmation du boîtier d'analyse et sortie du mode.

*Arrêt du contrôle en cours*

Il faut raccorder le boîtier amovible et appuyer 2 fois sur la touche P : l'affichage passe en rouge clignotant après 10 secondes. La friteuse pourra être réinitialisée après avoir été mise hors tension.

*Auto-diagnostic*

Trois procédures sont prévues pour s'assurer du bon fonctionnement du contrôleur :

* si les bornes de l'alimentation secteur ne sont pas correctement raccordées (inversion phase et neutre), le voyant vert clignote, le klaxon est mis en service et la friteuse ne peut pas être utilisée (problème pouvant survenir à l'installation du contrôleur),

* les voyants et le klaxon du module d'interface peuvent être vérifiés en appuyant sur le bouton poussoir seulement pendant la période de mise en chauffe de la friteuse (voyants vert et jaune allumés),

* si la sonde présente un défaut, les voyants rouge et jaune clignotent et le klaxon est mis en service. La procédure d'arrêt est la même que pour une détection d'huile usée.

*Marche forcée*

Le contrôleur peut être mis en marche forcée seulement si la sonde présente un défaut en programmant 255 dans la consigne n° 1 : le voyant jaune s'allume, la friteuse est remise en service mais sans contrôle de l'huile.

Pour sortir ce de mode, il faut raccorder le boîtier amovible et appuyer 2 fois sur la touche P. L'affichage passe alors en vert puis 10 secondes après :

- si la sonde a été réparée, en rouge clignotant. Le boîtier doit alors être réinitialisé,

- si la sonde présente toujours un défaut, continuité de l'affichage défaut sonde.

*Aides au dépannage*

Lorsque le boîtier amovible est raccordé au module d' interface, il affiche d'abord la fréquence du générateur permettant de juger rapidement du bon fonctionnement de la sonde. L'octet température huile usée est codé en fonction du type d'arrêt d'un contrôle.

| | |
|---|---|
| détection huile usée | = température du bain à ce moment-là |
| arrêt en cours | = 255 (arrêt volontaire) |
| coupure électrodes | = 254 (défaut sonde) |
| court-circuit électrodes | = 253 (défaut sonde) |
| coupure thermocouple | = 252 (défaut sonde) |
| jours max dépassés | = 251 (sécurité sur la durée maximale d'utilisation du même bain) |

Ces valeurs peuvent être visualisées par le boîtier amovible.

*Suivi de l'utilisation de la friteuse*

En appuyant sur la touche L du boîtier amovible raccordé au module d'interface, les informations mémorisées par le boîtier d'analyse sont transférées dans le boîtier amovible et peuvent être examinées par celui-ci :

*1) Affichage des paramètres du bain en cours d'utilisation*

Des pressions successives sur la touche N permettent de visualiser 8 valeurs :

n°1    12 = bain en cours
n°2    consigne de température programmée
n°3    consigne de dégradation programmée
n°4    température initiale
n°5    fréquence initiale
n°6    dernière valeur de la mesure de la température ou
       codage du type d'arrêt du contrôle
n°7    fréquence F usée à la détection
       (dernière valeur de la mesure de la fréquence)
n°8    nombre de jours d'utilisation du bain

*2) Affichage des paramètres des 11 derniers bains précédents*

Une pression sur la touche R repositionne l'affichage sur le paramètre n° 1 du bain précédent. Les 8 paramètres de ce bain sont accessibles en appuyant sur la touche N comme indiqué précédemment.

*3) Affichage des consignes, des seuils de détection et de l'état interne du contrôleur*

Une pression sur la touche P suivie de pressions sur la touche N permet d'accéder aux 8 valeurs suivantes :

n°1    consigne de température (réglable entre 0 et 254)
n°2    consigne de dégradation (réglable entre 0 et 255)
n°3    fréquence initiale du bain en cours
n°4    fréquence F usée du bain en cours calculée par le boîtier d'analyse
n°5    fréquence F 80% du bain en cours calculée par le boîtier d'analyse
n°6    état interne du boîtier d'analyse
n°7    nombre de mesures faites depuis la réinitialisation
n°8    adresse mémoire en cours

Le dispositif décrit ci-dessus est très complet, et combine plusieurs caractéristiques essentielles et accessoires d'un dispositif conforme à l'invention (qui peut donc être plus simple). Toutefois, on peut noter à son propos les avantages suivants :

- *un contrôle permanent de l'huile ou de la graisse :*

     la sonde de mesure est placée à demeure dans la cuve de cuisson et le dispositif d'analyse, d'asservissement et de signalisation est fixé à l'intérieur de la friteuse ;

- *un contrôle automatique :*

     après réinitialisation du dispositif de contrôle lors du remplissage de la cuve de cuisson avec de l'huile neuve, le contrôle de la dégradation ne nécessite aucune intervention humaine ;

- *un contrôle interdisant, après délai, l'utilisation de la friteuse avec - une huile ou une graisse usée :*

     grâce à un contact disponible sur le boîtier d'analyse, câblé en série avec l'alimentation du système de commande de la friteuse et qui s'ouvre 30 mn après la remise sous tension de la friteuse, une détection étant survenue avant la dernière mise hors tension de la friteuse et la réinitialisation du contrôleur n'ayant pas été faite dans cet intervalle de temps ;

- *un contrôle mettant en oeuvre un sonde capacitive :*

la sonde est principalement constituée de 2 électrodes métalliques isolées entre elles pour former un condensateur où l'huile ou la graisse à contrôler remplit l'espace inter-électrodes et constitue le diélectrique du condensateur ;

- *un contrôle où la sonde capacitive fait par exemple partie d'un oscillateur selon le schéma de principe fig. 9 :*

sa période d'oscillation T = aC où $\underline{a}$ est une constante fixée par les valeurs des résistances R, R1 et R2 et où $\underline{C}$ est une capacité constituée par la somme de deux capacités :

1°) une capacité $C_f$, fixe pour le contrôleur mais susceptible de varier d'un contrôleur à un autre, elle-même la somme de 2 capacités :

1.a) la capacité d'entrée de l'amplificateur opérationnel, et
1.b) la capacité du câble de liaison entre le contrôleur et la sonde.

2°) une capacité $C_s$ qui est la capacité de la sonde de mesure. Elle est égale à $\in_r$ x $C_{s\text{-air}}$ où $\in_r$ est la permittivité relative de l'huile et $C_{s\text{-air}}$ la capacité de la sonde mesurée dans le vide ou dans l'air.

- *un contrôle où une électrode de la sonde est au potentiel de la cuve de cuisson :*

l'oscillateur mis en oeuvre permet d'utiliser la cuve comme l'une des électrodes de la sonde pour simplifier sa réalisation et en diminuer le coût.

- *un contrôle basé sur la mesure de la variation absolue $\Delta T$ de la période T de l'oscillateur :*

la période T = a . ($C_f$+ $C_s$),
la permittivité relative de l'huile ou de la graisse neuve est notée $\in_m$ et celle de l'huile ou de la graisse usée notée $\in_{ru}$ avec la relation $\in_{ru} > \in_m$. La variation $\Delta T$ de la période T de l'oscillateur entre une huile ou une graisse neuve et une huile ou une graisse usée est :

$$\Delta T = T_u - T_n,$$

soit

$$\Delta T = a \times C_s \times (\in_{ru} - \in_m)$$

$\Delta T$ ne dépend pas de la capacité de $C_f$ . Cela conduit à une excellente interchangeabilité entre sondes et contrôleurs.

- *un contrôle dont les seuils de détection sont calculés à partir de $\Delta T$ :*

après réinitialisation lors d'un changement d'huile, le boîtier d'analyse détermine la fréquence $f_i$ à prendre en compte pour l'initialisation. Il calcule à partir de cette fréquence et de la consigne $\Delta T$ la période $T_{80\%}$ correspondant à une dégradation de 80 % et la période $T_{usée}$ correspondant à une huile usée, et en déduit les fréquences $F_{80\%}$ et Fusée qui sont les 2 seuils de détection du contrôle.

- *un contrôle dont la consigne $\Delta T$ est fixée expérimentalement par essais comparatifs avec la méthode d'analyse de référence normalisé :*

$\Delta T$ est une valeur absolue qui ne dépend que des caractéristiques géométriques de la sonde mesure, de la constante a du générateur et de la permittivité de l'huile ou de la graisse à contrôler.

- *un contrôle dont la fréquence $f_1$ prise en compte à l'initialisation pour le calcul des consignes est la fréquence maximale mesurée pendant les première heures d'utilisation du bain d'huile ou de graisse ;*

- *un contrôle dont la fréquence de l'oscillateur est telle que, pour la variation minimale ΔT à détecter, la variation de⁻ fréquence correspondante soit voisine de (100/p) en Hz où p représente la précision en % de la mesure ;*
- *un contrôle où un capteur de température est incorporé à la sonde :*
- *un contrôle dont la consigne de température permet d'inhiber les mesure lorsque la température de l'huile ou de la graisse est inférieure à la consigne ;*
- *un contrôle dont les consignes ne sont programmables qu'avec un boîtier d'un boîtier extérieur ;*
- *un contrôle dont la programmation des consignes n'est possible que pendant la demi-heure qui suit un changement d'huile,* disposition qui empêche toute modification de celles-ci pendant l'utilisation d'un bain ;
- *un contrôle dont la remise en service de la friteuse après ouverture du contact d'asservissement du boîtier d'analyse n'est possible qu'avec un boîtier extérieur;*
- *une fixation de la sonde où les électrodes sont verticales, pour diminuer leur encrassement ;*
- *l'implantation de l'oscillateur au plus près de la sonde* :

    disposition qui permet d'obtenir les possibilités de détection maximales avec une électronique économique d'analyse ;

- *un auto-diagnostic* qui signale à l'utilisateur tout défaut de la sonde ou du capteur de température ;
- *un module d'interface* séparé comprenant les trois voyants nécessaires à la visualisation du niveau d'usure de l'huile ou de la graisse et au contrôle de la sonde et du capteur de température, l'avertisseur sonore ou le klaxon, le connecteur de raccordement au boîtier amovible, le bouton poussoir test ou arrêt klaxon et test voyants, qui permet d'être implanté facilement dans la friteuse, à un endroit approprié, dans un espace restreint.

On peut noter que le dispositif de l'invention a une consommation très modérée. Même lorsque l'oscillateur est excité en permanence, le contrôleur a typiquement une consommation de l'ordre de 2 W, ce qui est très faible par rapport à la puissance installée (typiquement de l'ordre de 20 kW). Il n'est donc pas nécessaire (quoique possible) de prévoir des phases de réduction de consommation.

## Revendications

1. Procédé de contrôle d'une cuve de friture contenant un bain d'huile ou de graisse de friture destiné à suivre une pluralité de cycles de chauffe, selon lequel :

    * on installe à demeure dans une zone de la cuve adaptée à être immergée dans le bain une sonde capacitive (1) dont l'espace diélectrique est constitué par une portion dudit bain, et, après remplissage (E1) de la cuve avec ce bain, on déclenche une procédure d'initialisation (E2) au cours de laquelle on mesure une valeur initiale d'une caractéristique représentative de la constante diélectrique de ce bain en conditions de chauffe, et on fixe un critère de mise hors service,
    * de façon automatique, on détecte (E3), de manière répétée au cours des cycles de chauffe suivants, une mesure de cette caractéristique et on déclenche (E6) une procédure de mise hors service lorsqu'il est détecté (E4) que le critère de mise hors service est satisfait.

2. Procédé selon la revendication 1, **caractérisé en ce que** on mesure périodiquement la valeur instantanée de ladite caractéristique au moins une fois par heure.

3. Procédé selon la revendication 1 ou la revendication 2, **caractérisé en ce que** les mesures sont déduites de la moyenne de plusieurs mesures instantanées réparties sur la durée séparant deux détections de mesure.

4. Procédé selon l'une quelconque des revendications 1 à 3, **caractérisé en ce que** ce contrôle est continu ou quasi-continu, sans intervention humaine.

5. Procédé selon l'une quelconque des revendications 1 à 4, **caractérisé en ce que** l'on mesure ladite caractéristique par excitation d'un circuit oscillant dont fait partie la sonde capacitive, et ladite caractéristique est la période d'oscillation du circuit oscillant.

6. Procédé selon l'une quelconque des revendications 1 à 5, **caractérisé en ce que** le critère de mise hors service est le franchissement d'un seuil de consigne par la différence entre la valeur instantanée et la valeur initiale de ladite caractéristique.

**7.** Procédé selon l'une quelconque des revendications 1 à 5, **caractérisé en ce qu'**on fixe en outre un critère d'alerte et on déclenche (E5) une procédure d'alerte lorsqu'il est détecté que ce critère d'alerte est satisfait.

**8.** Procédé selon la revendication 7, **caractérisé en ce qu'**on fixe en outre un critère d'alerte en fonction de la valeur initiale de la caractéristique, défini par un seuil intermédiaire de différence entre les valeurs initiale et instantanée de la caractéristique qui est inférieure à la différence définissant le critère de mise hors service, et on déclenche (E5) une procédure d'alerte lorsque ce seuil intermédiaire est franchi.

**9.** Procédé selon l'une quelconque des revendications 1 à 8, **caractérisé en ce que** l'on inhibe la procédure de mise hors service jusqu'au cycle de chauffé suivant celui au cours duquel le critère de mise hors service a été détecté.

**10.** Procédé selon la revendication 9, **caractérisé en ce que** la procédure de mise hors service consiste en un arrêt du cycle de chauffe qui suit celui au cours duquel le critère de mise hors service a été détecté, après un temps prédéterminé.

**11.** Procédé selon l'une quelconque des revendications 1 à 10, **caractérisé en ce que** l'on détecte en outre une caractéristique représentative de la température du bain, et on inhibe les mesures de la caractéristique ou le contrôle du critère de mise hors service lorsque la température du bain est inférieure à un seuil minimum de température.

**12.** Procédé selon l'une quelconque des revendications 1 à 11, **caractérisé en ce que** on mémorise des données concernant des bains précédents.

**13.** Procédé selon la revendication 12, **caractérisé en ce que**, lorsqu'on détecte que, après un temps prédéterminé après le début d'un cycle de chauffe suivant un cycle de chauffe au cours duquel le critère de mise hors service a été constaté, il n'y a pas eu de procédure d'initialisation, on provoque l'interruption du cycle de chauffe.

**14.** Procédé selon la revendication 12 ou la revendication 13, **caractérisé en ce que** l'on inhibe toute procédure d'initialisation déclenchée au-delà d'un délai prédéterminé après le début d'un cycle de chauffe suivant un cycle de chauffe au cours duquel le critère de mise hors service a été constaté.

**15.** Procédé selon l'une quelconque des revendications 12 à 14, **caractérisé en ce que** l'on définit la procédure d'initialisation en fonction d'au moins l'une des procédures d'initialisation des bains précédents mémorisées.

**16.** Procédé selon l'une quelconque des revendications 1 à 15, **caractérisé en ce que** la procédure d'initialisation n'est déclenchable qu'à partir d'un boîtier indépendant de la cuve.

**17.** Dispositif de contrôle automatique d'une cuve de friture contenant un bain d'huile ou de graisse de friture destiné à suivre une pluralité de cycles de chauffe, comportant :

* une sonde capacitive (1) installée à demeure dans une zone de la cuve adaptée à être immergée dans le bain
* une unité de mesure et de traitement (2, 6) connectée à la sonde et à l'alimentation électrique de la cuve adaptée à mesurer (E3) des valeurs d'une caractéristique représentative de la constante diélectrique de ce bain en conditions de chauffe, et conçue en sorte

    - de mémoriser (E2), en réponse à un ordre d'initialisation, une mesure initiale de cette caractéristique et un critère de mise hors service défini en fonction de cette valeur initiale, et
    - de détecter de manière périodique au cours des cycles de chauffe du bain une mesure de cette caractéristique, et de déclencher une procédure de mise hors service lorsqu'il est détecté que le critère de mise hors service est satisfait par cette mesure de la caractéristique,

* une unité d'initialisation adaptée à être connectée à l'unité de mesure et de traitement et à lui transmettre un ordre d'initialisation et des données d'initialisation concernant le critère de mise hors service.

**18.** Dispositif selon la revendication 17, **caractérisé en ce que** l'unité de mesure et de traitement est conçue en sorte de mesurer ladite caractéristique au moins une fois par heure.

**19.** Dispositif selon la revendication 17 ou la revendication 18, **caractérisé en ce que** l'unité de mesure et de traitement est conçue en sorte de déduire la mesure de la caractéristique à partir de plusieurs mesures instantanées successives réparties sur la durée séparant deux détections de mesures.

20. Dispositif selon l'une quelconque des revendications 17 à 19, **caractérisé en ce que** l'unité de mesure et de traitement est conçue en sorte de mesurer ladite caractéristique de façon continue ou quasi-continue.

21. Dispositif selon l'une quelconque des revendications 17 à 20, **caractérisé en ce que** la sonde est montée dans un circuit oscillant, et l'unité de mesure et de traitement est conçue en sorte de mesurer la période d'oscillation du circuit oscillant.

22. Dispositif selon l'une quelconque des revendications 17 à 21, **caractérisé en ce que** l'unité de mesure et de traitement est conçue en sorte de pouvoir détecter le franchissement d'un seuil de consigne défini par l'unité d'initialisation au sein des données concernant le critère de mise hors service, par la différence entre la mesure détectée et la valeur initiale de ladite caractéristique.

23. Dispositif selon l'une quelconque des revendications 17 à 22, **caractérisé en ce que** l'unité de mesure et de traitement est en outre conçue en sorte de mémoriser un critère d'alerte défini par l'unité d'initialisation, et de déclencher (E5) une procédure d'alerte lorsqu'il est détecté que ce critère d'alerte est satisfait par la mesure détectée de la caractéristique.

24. Dispositif selon l'une quelconque des revendications 17 à 23, **caractérisé en ce que** l'unité de mesure et de traitement est conçue en sorte d'inhiber la procédure de mise hors service jusqu'au cycle de chauffe suivant celui au cours duquel le critère de mise hors service a été détecté.

25. Dispositif selon la revendication 24, **caractérisé en ce que** la procédure de mise hors service consiste en un arrêt, par coupure de l'alimentation de la cuve, du cycle de chauffe qui suit celui au cours duquel le critère de mise hors service a été détecté, après un temps prédéterminé.

26. Dispositif selon l'une quelconque des revendications 17 à 25. **caractérisé en ce qu'**il comporte en outre un élément détecteur (21, 22) pour mesurer une caractéristique représentative de la température du bain, et l'unité de mesure et de traitement est connectée à cet élément détecteur et est conçue en sorte d'inhiber les mesures de la caractéristique ou le contrôle du critère de mise hors service lorsque la température du bain est inférieure à un seuil minimum de température.

27. Dispositif selon l'une quelconque des revendications 17 à 26, **caractérisé en ce que** l'unité de mesure et de traitement est en outre conçue en sorte de mémoriser des données concernant des bains précédents.

28. Dispositif selon la revendication 27, **caractérisé en ce que** l'unité de mesure et de traitement est conçue en sorte de pouvoir détecter que, après un temps prédéterminé après le début d'un cycle de chauffe suivant un cycle de chauffe au cours duquel le critère de mise hors service a été constaté, il n'y a pas eu de transmission d'ordre d'initialisation ou de données depuis l'unité d'initialisation, et de pouvoir alors provoquer l'interruption du cycle de chauffe par coupure de l'alimentation de la cuve.

29. Dispositif selon la revendication 27 ou la revendication 28, **caractérisé en ce que** l'unité de mesure et de traitement est en outre conçue en sorte d'inhiber tout ordre ou donnée d'initialisation en provenance de l'unité d'initialisation, au-delà d'un délai prédéterminé après le début d'un cycle de chauffe suivant un cycle de chauffe au cours duquel le critère de mise hors service a été constaté.

30. Dispositif selon l'une quelconque des revendications 27 à 29, **caractérisé en ce que** l'unité d'initialisation est conçue en sorte de définir les données d'initialisation en fonction des données d'initialisation et de données échangées avec l'unité de mesure et de traitement pour l'un au moins des bains précédents.

31. Dispositif selon l'une quelconque des revendications 17 à 30, **caractérisé en ce que** l'unité de mesure et de traitement est fixée à la cuve, et l'unité d'initialisation est contenue dans un boîtier amovible.

32. Dispositif selon l'une quelconque des revendications 17 à 31, **caractérisé en ce que** la sonde capacitive est fixée dans la cuve en dessous de résistances dont la cuve est munie pour le chauffage du bain, cette cuve faisant partie d'une friteuse electrique.

**33.** Dispositif selon l'une quelconque des revendications 17 à 32, **caractérisé en ce que** la sonde capacitive comporte deux électrodes dont l'une est au potentiel constant de la cuve.

**34.** Dispositif selon la revendication 33, **caractérisé en ce que** la cuve est utilisée comme l'une des électrodes de la sonde.

**Patentansprüche**

**1.** Verfahren zum Steuern eines Frittierbehälters, der ein Bad aus Frittieröl oder -fett enthält, das dazu vorgesehen ist, mehrere Heizzyklen zu durchlaufen, bei dem:

- in einer Zone des Behälters, die in das Bad eingetaucht werden kann, eine kapazitive Sonde (1) ständig installiert wird, deren dielektrischer Zwischenraum durch einen Teil des Bades gebildet wird, und nach dem Befüllen (E1) des Behälters mit diesem Bad eine Initialisierungsprozedur (E2) gestartet wird, in deren Verlauf ein Anfangswert einer Charakteristik gemessen wird, die für die Dielektrizitätskonstante dieses Bades unter Heizbedingungen repräsentativ ist, und ein Abschaltkriterium festgelegt wird, - während folgender Heizzyklen wiederholt automatisch ein Messwert dieser Charakteristik erfasst wird (E3) und eine Abschaltprozedur ausgelöst wird (E6), wenn festgestellt wird (E4), dass das Abschaltkriterium erfüllt ist.

**2.** Verfahren nach Anspruch 1, **dadurch gekennzeichnet, dass** der aktuelle Wert der Charakteristik wenigstens einmal pro Stunde periodisch gemessen wird.

**3.** Verfahren nach Anspruch 1 oder Anspruch 2, **dadurch gekennzeichnet, dass** die Messwerte aus dem Mittelwert mehrerer aktueller Messwerte abgeleitet werden, die über eine Zeitdauer verteilt sind, die zwei Erfassungen von Messwerten trennt.

**4.** Verfahren nach einem der Ansprüche 1 bis 3, **dadurch gekennzeichnet, dass** diese Steuerung kontinuierlich oder fast kontinuierlich ohne menschlichen Eingriff ist.

**5.** Verfahren nach einem der Ansprüche 1 bis 4, **dadurch gekennzeichnet, dass** die Charakteristik durch Erregen einer Oszillatorschaltung, wovon die kapazitive Sonde einen Teil bildet, gemessen wird und die Charakteristik die Oszillationsperiode der Oszillatorschaltung ist.

**6.** Verfahren nach einem der Ansprüche 1 bis 5, **dadurch gekennzeichnet, dass** das Abschaltkriterium das Überschreiten eines Stell-Schwellenwertes durch die Differenz zwischen dem aktuellen Wert und dem Anfangswert der Charakteristik ist.

**7.** Verfahren nach einem der Ansprüche 1 bis 5, **dadurch gekennzeichnet, dass** außerdem ein Warnkriterium festgelegt wird und eine Warnprozedur gestartet wird (E5), wenn festgestellt wird, dass dieses Warnkriterium erfüllt ist.

**8.** Verfahren nach Anspruch 7, **dadurch gekennzeichnet, dass** außerdem ein Warnkriterium in Abhängigkeit vom Anfangswert der Charakteristik festgelegt wird, das durch einen Zwischenschwellenwert der Differenz zwischen dem Anfangswert und dem aktuellen Wert der Charakteristik, die kleiner als die das Abschaltkriterium definierende Differenz ist, definiert ist, und eine Warnprozedur ausgelöst wird (E5), wenn dieser Zwischenschwellenwert überschritten wird.

**9.** Verfahren nach einem der Ansprüche 1 bis 8, **dadurch gekennzeichnet, dass** die Abschaltprozedur bis zu dem Heizzyklus verhindert wird, der jenem folgt, in dessen Verlauf das Abschaltkriterium erfasst worden ist.

**10.** Verfahren nach Anspruch 9, **dadurch gekennzeichnet, dass** die Abschaltprozedur darin besteht, den Heizzyklus, der jenem folgt, in dessen Verlauf das Abschaltkriterium erfasst worden ist, nach einer vorgegebenen Zeit anzuhalten.

**11.** Verfahren nach einem der Ansprüche 1 bis 10, **dadurch gekennzeichnet, dass** außerdem eine die Temperatur des Bades angebende Charakteristik erfasst wird und die Messungen der Charakteristik oder die Steuerung des Abschaltkriteriums verhindert wird, wenn die Temperatur des Bades niedriger als ein unterster Schwellenwert der Temperatur ist.

**12.** Verfahren nach einem der Ansprüche 1 bis 11, **dadurch gekennzeichnet, dass** die Daten, die die vorhergehenden Bäder betreffen, gespeichert werden.

**13.** Verfahren nach Anspruch 12, **dadurch gekennzeichnet, dass** dann, wenn nach einer vorgegebenen Zeit seit dem Beginn eines Heizzyklus, der einem Heizzyklus folgt, in dessen Verlauf das Abschaltkriterium festgestellt worden ist, erfasst wird, dass keine Initialisierungsprozedur stattgefunden hat, die Unterbrechung des Heizzyklus bewirkt wird.

**14.** Verfahren nach Anspruch 12 oder Anspruch 13, **dadurch gekennzeichnet, dass** jede Initialisierungsprozedur, die jenseits einer vorgegebenen Verzögerung seit dem Beginn eines Heizzyklus, der einem Heizzyklus folgt, in dessen Verlauf das Abschaltkriterium festgestellt worden ist, gestartet wird, verhindert wird.

**15.** Verfahren nach einem der Ansprüche 12 bis 14, **dadurch gekennzeichnet, dass** die Initialisierungsprozedur in Abhängigkeit von wenigstens einer der Initialisierungsprozeduren der gespeicherten vorhergehenden Bäder definiert wird.

**16.** Verfahren nach einem der Ansprüche 1 bis 15, **dadurch gekennzeichnet dass** die Initialisierungsprozedur nur anhand eines von dem Behälter unabhängigen Gehäuses auslösbar ist.

**17.** Vorrichtung zum automatischen Steuern eines Frittierbehälters, der ein Bad aus Frittieröl oder -fett enthält, das mehrere Heizzyklen durchlaufen soll, mit:

- einer kapazitiven Sonde (1), die ständig in einer Zone des Behälters, die in das Bad eingetaucht werden kann, installiert ist,
- einer Mess- und Verarbeitungseinheit (2, 6), die mit der Sonde und mit der elektrischen Versorgung des Behälters verbunden ist und Werte einer die Dielektrizitätskonstante dieses Bades unter Heizbedingungen repräsentierenden Charakteristik messen kann (E3) und so entworfen ist, dass sie

  - in Reaktion auf einen Initialisierungsbefehl einen Anfangsmesswert dieser Charakteristik und ein in Abhängigkeit von diesem Anfangsmesswert definiertes Abschaltkriterium speichert (E2) und
  - während der Heizzyklen des Bades periodisch einen Messwert dieser Charakteristik erfasst und eine Abschaltprozedur startet, wenn festgestellt wird, dass dieser Messwert der Charakteristik das Abschaltkriterium erfüllt,

- einer Initialisierungseinheit, die mit der Mess- und Verarbeitungseinheit verbunden werden kann und an sie einen Initialisierungsbefehl sowie das Abschaltkriterium betreffende Initialisierungsdaten übertragen kann.

**18.** Vorrichtung nach Anspruch 17, **dadurch gekennzeichnet, dass** die Mess- und Verarbeitungseinheit so beschaffen ist, dass sie die Charakteristik wenigstens einmal pro Stunde misst.

**19.** Vorrichtung nach Anspruch 17 oder Anspruch 18, **dadurch gekennzeichnet, dass** die Mess- und Verarbeitungseinheit so entworfen ist, dass sie den Messwert der Charakteristik aus mehreren aufeinander folgenden aktuellen Messungen ableitet, die über eine Zeitdauer verteilt sind, die zwei Erfassungen von Messungen trennt.

**20.** Vorrichtung nach einem der Ansprüche 17 bis 19, **dadurch gekennzeichnet, dass** die Mess- und Verarbeitungseinheit so entworfen ist, dass die Charakteristik kontinuierlich oder fast kontinuierlich gemessen wird.

**21.** Vorrichtung nach einem der Ansprüche 17 bis 20, **dadurch gekennzeichnet, dass** die Sonde in einer Oszillatorschaltung angebracht ist und die Mess- und Verarbeitungseinheit so entworfen ist, dass sie die Oszillationsperiode der Oszillatorschaltung misst.

**22.** Vorrichtung nach einem der Ansprüche 17 bis 21, **dadurch gekennzeichnet, dass** die Mess- und Verarbeitungseinheit so entworfen ist, dass sie das Überschreiten eines durch die Initialisierungseinheit anhand der das Abschaltkriterium betreffenden Daten definierten Stell-Schwellenwertes durch die Differenz zwischen dem erfassten Messwert und dem Anfangswert der Charakteristik erfassen kann.

**23.** Vorrichtung nach einem der Ansprüche 17 bis 22, **dadurch gekennzeichnet, dass** die Mess- und Verarbeitungseinheit außerdem so entworfen ist, dass sie ein Warnkriterium speichert, das durch die Initialisierungseinheit definiert ist, und eine Warnprozedur auslöst (E5), wenn festgestellt wird, dass das Warnkriterium von dem erfassten Messwert der Charakteristik erfüllt wird.

24. Vorrichtung nach einem der Ansprüche 17 bis 23, **dadurch gekennzeichnet, dass** die Mess- und Verarbeitungseinheit so entworfen ist, dass sie die Abschaltprozedur bis zu dem Heizzyklus verhindert, der jenem folgt, in dessen Verlauf das Abschaltkriterium erfasst worden ist.

25. Vorrichtung nach Anspruch 24, **dadurch gekennzeichnet, dass** die Abschaltprozedur darin besteht, nach einer vorgegebenen Zeitdauer den Heizzyklus, der jenem folgt, in dessen Verlauf das Abschaltkriterium erfasst worden ist, durch Unterbrechen der Versorgung des Behälters anzuhalten.

26. Vorrichtung nach einem der Ansprüche 17 bis 25, **dadurch gekennzeichnet, dass** sie außerdem ein Detektorelement (21, 22) zum Messen einer die Temperatur des Bades repräsentierenden Charakteristik umfasst und die Mess- und Verarbeitungseinheit mit diesem Detektorelement verbunden und so entworfen ist, dass sie die Messungen der Charakteristik oder die Steuerung des Abschaltkriteriums verhindert, wenn die Temperatur des Bades unter einem kleinsten Temperaturschwellenwert liegt.

27. Vorrichtung nach einem der Ansprüche 17 bis 26, **dadurch gekennzeichnet, dass** die Mess- und Verarbeitungseinheit außerdem so entworfen ist, dass sie Daten, die vorhergehende Bäder betreffen, speichert.

28. Vorrichtung nach Anspruch 27, **dadurch gekennzeichnet, dass** die Mess- und Verarbeitungseinheit so entworfen ist, dass sie feststellen kann, dass nach einer vorgegebenen Zeit seit dem Beginn eines Heizzyklus, der jenem Heizzyklus folgt, in dessen Verlauf das Abschaltkriterium festgestellt worden ist, keine Übertragung eines Initialisierungsbefehls oder von Daten von der Initialisierungseinheit stattgefunden hat, und dann die Unterbrechung des Heizzyklus durch Unterbrechen der Versorgung des Behälters hervorrufen kann.

29. Vorrichtung nach Anspruch 27 oder Anspruch 28, **dadurch gekennzeichnet, dass** die Mess- und Verarbeitungseinheit außerdem so entworfen ist, dass sie jeden Initialisierungsbefehl und alle Initialisierungsdaten, die von der Initialisierungseinheit stammen, jenseits einer vorgegebenen Verzögerung seit dem Beginn eines Heizzyklus, der jenem Heizzyklus folgt, in dessen Verlauf das Abschaltkriterium festgestellt worden ist, verhindern kann.

30. Vorrichtung nach einem der Ansprüche 27 bis 29, **dadurch gekennzeichnet, dass** die Initialisierungseinheit so entworfen ist, dass sie Initialisierungsdaten in Abhängigkeit von Initialisierungsdaten und von Daten, die mit der Mess- und Verarbeitungseinheit für wenigstens eines der vorhergehenden Bäder ausgetauscht werden, definiert.

31. Vorrichtung nach einem der Ansprüche 17 bis 30, **dadurch gekennzeichnet, dass** die Mess- und Verarbeitungseinheit in dem Behälter befestigt ist und die Initialisierungseinheit in einem abnehmbaren Gehäuse enthalten ist.

32. Vorrichtung nach einem der Ansprüche 17 bis 31, **dadurch gekennzeichnet, dass** die kapazitive Sonde in dem Behälter unterhalb von ohmschen Widerständen, mit denen der Behälter versehen ist, um das Bad zu erhitzen, befestigt ist, wobei dieser Behälter einen Teil einer elektrischen Frittiereinrichtung bildet.

33. Vorrichtung nach einem der Ansprüche 17 bis 32, **dadurch gekennzeichnet, dass** die kapazitive Sonde zwei Elektroden umfasst, wovon eine auf dem konstanten Potential des Behälters liegt.

34. Vorrichtung nach Anspruch 33, **dadurch gekennzeichnet, dass** der Behälter als eine der Elektroden der Sonde verwendet wird.

## Claims

1. Process for monitoring a frying vat containing a bath of frying oil or fat intended to follow a plurality of heating cycles, according to which:

   * in a zone of the vat suitable for immersion in the bath, a capacitive probe (1) is permanently installed, the dielectric space of which is constituted by a portion of said bath and, after filling (E1) of the vat with this bath, an initialization procedure (E2) is triggered during which an initial value of a characteristic representative of the dielectric constant of this bath in heating conditions is measured, and a shutdown criterion is fixed,

&ast; automatically, a measurement of this characteristic is repeatedly detected (E3) during the following heating cycles, and a shutdown procedure is triggered (E6) when it is detected (E4) that the shutdown criterion is met.

2. Process according to claim 1, **characterized in that** the instantaneous value of said characteristic is periodically measured at least once an hour.

3. Process according to claim 1 or claim 2, **characterized in that** the measurements are deduced from the average of several instantaneous measurements spread over the period separating two measurement detections.

4. Process according to any one of claims 1 to 3, **characterized in that** this monitoring is continuous or virtually continuous without human intervention.

5. Process according to any one of claims 1 to 4, **characterized in that** said characteristic is measured by excitation of an oscillating circuit of which the capacitive probe forms part, and said characteristic is the oscillation period of the oscillating circuit.

6. Process according to any one of claims 1 to 5, **characterized in that** the shutdown criterion is the exceeding of a set value threshold by the difference between the instantaneous value and the initial value of said characteristic.

7. Process according to any one of claims 1 to 5, **characterized in that** an alert criterion is also fixed and an alert procedure is triggered (E5) when it is detected that this alert criterion is met.

8. Process according to claim 7, **characterized in that** an alert criterion is also fixed in relation to the initial value of the characteristic, defined by an intermediate threshold of the difference between the initial and instantaneous values of the characteristic which is less than the difference defining the shutdown criterion, and an alert procedure is triggered (E5) when this intermediate threshold is exceeded.

9. Process according to any one of claims 1 to 8, **characterized in that** the shutdown procedure is inhibited until the heating cycle following that during which the shutdown criterion has been detected.

10. Process according to claim 9, **characterized in that** the shutdown procedure consists of a stoppage of the heating cycle which follows that during which the shutdown criterion has been detected, after a predetermined time.

11. Process according to any one of claims 1 to 10, **characterized in that** a characteristic representative of the temperature of the bath is also detected, and the measurements of the characteristic or the monitoring of the shutdown criterion are inhibited when the temperature of the bath is less than a minimum temperature threshold.

12. Process according to any one of claims 1 to 11, **characterized in that** data relating to previous baths are stored.

13. Process according to claim 12, **characterized in that**, when it is detected that, after a predetermined time after the start of a heating cycle following a heating cycle during which the shutdown criterion has been established, no initialization procedure took place, the heating cycle is interrupted.

14. Process according to claim 12 or claim 13, **characterized in that** any initialization procedure triggered after a predetermined period after the start of a heating cycle following a heating cycle during which the shutdown criterion has been established is inhibited.

15. Process according to any one of claims 12 to 14, **characterized in that** the initialization procedure is defined in relation to at least one of the stored initialization procedures of the previous baths.

16. Process according to any one of claims 1 to 15, **characterized in that** the initialization procedure can be triggered only from a box independent of the vat.

17. Device for the automatic monitoring of a frying vat containing a bath of frying oil or fat intended to follow a plurality of heating cycles, comprising:

&ast; a capacitive probe (1) permanently installed in a zone of the vat suitable for immersion in the bath
&ast; a measurement and processing unit (2,6) connected to the probe and to the electricity supply of the vat, suitable for the measurement (E3) of values of a characteristic representative of the dielectric constant of this bath in heating conditions, and conceived so as to

- store (E2), in response to an initialization command, an initial value of this characteristic and a shutdown criterion defined in relation to this initial value, and
- detect periodically during the heating cycles of the bath, a measurement of this characteristic, and to trigger a shutdown procedure when it is detected that the shutdown criterion is met by this measurement of the characteristic,

* an initialization unit suitable for connection to the measurement and processing unit and for transmission to it of an initialization command and initialization data concerning the shutdown criterion.

18. Device according to claim 17, **characterized in that** the measurement and processing unit is conceived so as to measure said characteristic at least once an hour.

19. Device according to claim 17 or claim 18, **characterized in that** the measurement and processing unit is conceived so as to deduce the measurement of the characteristic from several successive instantaneous measurements spread over the period separating two measurement detections.

20. Device according to any one of claims 17 to 19, **characterized in that** the measurement and processing unit is conceived so as to measure said characteristic in a continuous or virtually continuous manner.

21. Device according to any one of claims 17 to 20, **characterized in that** the probe is fitted in an oscillating circuit, and the measurement and processing unit is conceived so as to measure the oscillation period of the oscillating circuit.

22. Device according to any one of claims 17 to 21, **characterized in that** the measurement and processing unit is conceived so as to be able to detect the exceeding of a set value threshold defined by the initialization unit within the data relating to the shutdown criterion, by the difference between the detected measurement and the initial value of said characteristic.

23. Device according to any one of claims 17 to 22, **characterized in that** the measurement and processing unit is also conceived so as to store an alert criterion defined by the unit of initialization and to trigger (E5) an alert procedure when it is detected that this alert criterion is satisfied by the detected measurement of the characteristic.

24. Device according to any one of claims 17 to 23, **characterized in that** the measurement and processing unit is conceived so as to inhibit the shutdown procedure until the heating cycle following that during which the shutdown criterion has been detected.

25. Device according to claim 24, **characterized in that** the shutdown procedure consists of a stoppage, by cutting off the supply to the vat, of the heating cycle which follows that during which the shutdown criterion has been detected, after a predetermined time.

26. Device according to any one of claims 17 to 25, **characterized in that** it also contains a detection element (21, 22) for measuring a characteristic representative of the temperature of the bath, and the measurement and processing unit is connected to this detection element and is conceived so as to inhibit the measurements of the characteristic or the monitoring of the shutdown criterion when the temperature of the bath is lower than a minimum temperature threshold.

27. Device according to any one of claims 17 to 26, **characterized in that** the measurement and processing unit is also conceived so as to store data relating to previous baths.

28. Device according to claim 27, **characterized in that** the measurement and processing unit is conceived so as to be able to detect that, after a predetermined time after the start of a heating cycle following a heating cycle during which the shutdown criterion has been established, there has been no transmission of an initialization command or of data from the initialization unit, and to be able to then trigger the interruption of the heating cycle by cutting the supply to the vat.

**29.** Device according to claim 27 or claim 28, **characterized in that** the measurement and processing unit is also conceived so as to inhibit any command or initialization data from the initialization unit, after a predetermined period after the start of a heating cycle following a heating cycle during which the shutdown criterion has been established.

**30.** Device according to any one of claims 27 to 29, **characterized in that** the initialization unit is conceived so as to define the initialization data in relation to the initialization data and data exchanged with the measurement and processing unit for at least one of the previous baths.

**31.** Device according to any one of claims 17 to 30, **characterized in that** the measurement and processing unit is fixed to the vat, and the initialization unit is contained in a detachable box.

**32.** Device according to any one of claims 17 to 31, **characterized in that** the capacitive probe is fixed in the vat below resistors with which the vat is fitted for the heating of the bath, this vat being part of the electric deep fryer.

**33.** Device according to any one of claims 17 to 32, **characterized in that** the capacitive probe comprises two electrodes one of which is at the constant potential of the vat.

**34.** Device according to claim 33, **characterized in that** the vat is used as one of the electrodes of the probe.

Fig. 1

Fig. 2

Fig. 3

Fig. 4

Fig. 5

Fig. 6

Fig. 7

Fig. 8

Fig. 9

Fig. 10